# EUROPÄISCHE PATENTANMELDUNG

(11) **EP 1 201 203 A1**
(43) Veröffentlichungstag der Anmeldung: **02.05.2002**
(21) Anmeldenummer: 00123194.3
(22) Anmeldetag: 26.10.2000
(51) Int. Cl.: A61F 2/06

(54) **Vorrichtung zur Sondierung von Gefässabzweigungen**

(71) Anmelder: Jostra AG, 72145 Hirrlingen (DE)
(72) Erfinder: Stumpp, Gerd, 72145 Hirrlingen (DE); Klietsch, Dietmar, 71083 Herrenberg (DE)
(74) Vertreter: Möbus, Daniela, Dr.-Ing.

(57) **Zusammenfassung**

Eine Vorrichtung (10) zur Sondierung von Gefäßabzweigungen mit einem Katheterrohr (11), in dem ein erster Führungsdraht (13), an dem im Abstand zu seiner Spitze ein zweiter Führungsdraht (14) befestigt ist, geführt ist, wobei die beiden Führungsdrähte (13, 14) im entspannten Zustand einen spitzen Winkel einschließen und wobei das Katheterrohr (11) eine seitliche öffnung (17) aufweist, aus der beim Zurückziehen der Führungsdrähte (13, 14) die Spitze (16) des zweiten Führungsdrahts (14) austreten kann, und wobei auf der der Katheterrohrspitze abgewandten Seite der seitlichen öffnung (17) im Katheterrohr (11) ein Führungskörper (18) angeordnet ist, der eine Rampe (19) bildet, die die Spitze (16) des zweiten Führungsdrahts (14) beim Zurückziehen der Führungsdrähte (13, 14) durch die seitliche öffnung (17) nach außen leitet.

## Beschreibung

Die Erfindung betrifft eine Vorrichtung zur Sondierung von Gefäßabzweigungen, insbesondere zur Platzierung von Gefäßprothesen an Gefäßverzweigungsstellen, mit einem Katheterrohr, in dem ein erster Führungsdraht, an dem im Abstand zu seiner Spitze ein zweiter Führungsdraht befestigt ist, geführt ist, wobei die beiden Führungsdrähte im entspannten Zustand einen spitzen Winkel einschließen und wobei das Katheterrohr eine seitliche öffnung aufweist, aus der beim Zurückziehen der Führungsdrähte die Spitze des zweiten Führungsdrahts austreten kann.

Eine solche Hilfsvorrichtung ist im deutschen Gebrauchsmuster DE 298 22 381 U1 für die spezielle Anwendung des Einsetzens einer Aorten-Endoprothese beschrieben. Aorten-Endoprothesen werden insbesondere bei der Behandlung von Bauchaorten-Aneurysmen eingesetzt. Insgesamt wird dabei eine Y-förmige Prothese im Bereich der Verzweigung der Aorta in die beiden Beckenarterien im Rahmen einer minimal-invasiven chirurgischen Behandlung eingesetzt. Dafür wird mittels Kathetertechnik eine selbstexpandierende Gefäßprothese über die Arteria femoralis bis in die Aorta hochgeschoben und jenseits des Aneurysma freigesetzt. Anschließend wird ein Verlängerungsschenkel dieser sich von der Aorta in eine Beckenarterie erstreckenden Hauptprothese durch die andere Beckenarterie bis zur Verzweigung hochgeführt und mit der Hauptprothese zusammengesetzt. Das Einführen des Verlängerungsschenkels in die Verbindung mit der Hauptprothese ist dabei ein relativ schwieriger und diffiziler Vorgang, bei dem die bekannte Hilfsvorrichtung eine Erleichterung bietet. Die beiden ein Y bildenden Führungsdrähte werden dazu gemeinsam im Katheterrohr durch eine Beckenarterie bis in die Aorta vorgeschoben und anschließend die Hauptprothese entlang des Katheterrohrs vorgeschoben und platziert. Anschließend werden die Führungsdrähte etwas zurückgezogen, wobei der zweite Führungsdraht durch die seitliche Öffnung im Katheterrohr austritt und damit in die zweite Beckenarterie eingeführt werden kann. Durch die zweite Beckenarterie wird anschließend ein weiterer Führungsdraht eingeschoben, der an seinem vorderen Ende mit einer Schlinge versehen ist. Diese Schlinge kann mit dem vorzugsweise hakenförmig gebogenen Ende des zweiten Führungsdrahts der bekannten Hilfsvorrichtung erfasst werden. Nun kann der Verlängerungsschenkel der Hauptprothese über die miteinander gekoppelten Führungsdrähte leicht zur Verbindungsstelle mit der Hauptprothese vorgeschoben und an dieser fixiert werden.

Bei der bekannten Prothese hat es sich jedoch immer wieder als relativ schwierig herausgestellt, in allen Fällen ein sicheres Ausfädeln der Spitze des zweiten Führungsdrahts durch die seitliche Öffnung im Katheterrohr beim Zurückziehen der Führungsdrähte zu gewährleisten.

Der vorliegenden Erfindung liegt die Aufgabe zugrunde, die bekannte Hilfsvorrichtung derart weiterzubilden, dass ein zuverlässiges Austreten des zweiten Führungsdrahts aus der seitlichen Öffnung im Katheterrohr gewährleistet ist.

Die Aufgabe wird mit einer Vorrichtung der eingangs genannten Art erfindungsgemäß dadurch gelöst, dass auf der der Katheterrohrspitze abgewandten Seite der seitlichen Öffnung im Katheterrohr ein Führungskörper angeordnet ist, der eine zum öffnungsrand hinführende Rampe bildet, die die Spitze des zweiten Führungsdrahts beim Zurückziehen der Führungsdrähte durch die seitliche öffnung nach außen leitet. Solange die beiden Führungsdrähte im Katheterrohr nach vorn geführt werden. liegen sie aufgrund des geringen Rohrdurchmessers nahezu parallel nebeneinander im Rohr. Beim Zurückziehen der Führungsdrähte gleitet die Spitze des zweiten Führungsdrahts auf die Rampe auf und wird von dieser durch die seitliche öffnung im Katheterrohr nach außen gedrückt. Die Führung der Spitze des zweiten Führungsdrahts aus der seitlichen öffnung heraus wird dabei noch dadurch erleichtert, dass die Spitze in der Regel hakenförmig gekrümmt ist. Diese Vorkrümmung, die beim Vorwärtstreiben der Führungsdrähte ausgerollt ist, tendiert sofort wieder zum Einrollen, sobald in radialer Richtung des Katheterrohrs im Bereich der seitlichen öffnung ausreichend Platz hierfür vorhanden ist.

Vorzugsweise kann der Führungskörper zwei getrennte Durchgangsöffnungen zum Durchtritt der beiden Führungsdrähte aufweisen. Hierdurch ist gewährleistet, dass beide Drähte sauber im Katheterrohr ohne gegenseitiges Verhaken geführt werden können. Die Spitze des zweiten Führungsdrahts, der viel kürzer als der erste Führungsdraht ist, verlässt jedoch die Durchgangsöffnung, sobald die Drähte weit genug nach vorn geschoben worden sind. Dies ermöglicht aufgrund des Aufspreizens der beiden Drähte beim Zurückziehen der Drähte das Aufgleiten der Spitze des zweiten Führungsdrahts auf die Rampe.

Weitere Vorteile ergeben sich, wenn der Führungskörper formschlüssig mit dem Katheter verbunden ist. Dadurch kann ein axiales Verschieben, das die Führungseigenschaften beeinträchtigen würde, ausgeschlossen werden.

Bei einer besonders bevorzugten Ausführungsform wird der Führungskörper aus einem Klebstoff gebildet, wobei der Klebstoff durch eine weitere seitliche Öffnung in der Wand des Katheterrohrs einbringbar sein kann, sodass er diese Öffnung ausfüllt und so eine formschlüssige Verbindung mit dem Katheterrohr eingeht. Diese Art der Fertigung des Führungskörpers ist besonders einfach und gleichzeitig zuverlässig, was die Vermeidung eines axialen Verschiebens des Führungskörpers betrifft.

Nachfolgend wird ein bevorzugtes Ausführungsbeispiel einer erfindungsgemäßen Vorrichtung und eine Anwendungsmöglichkeit anhand der Zeichnung näher beschrieben.

### Es zeigen:

- Fig. 1: eine Seitenansicht einer erfindungsgemäßen Vorrichtung;
- Fig. 2: einen vergrößerten Teillängschnitt durch die Vorrichtung aus Fig. 1;
- Fig. 3: einen Querschnitt durch die Anordnung aus Fig. 2 entlang der Linie III-III;
- Fig. 4: eine schematische Ansicht einer Anwendung der Vorrichtung aus Fig. 1.

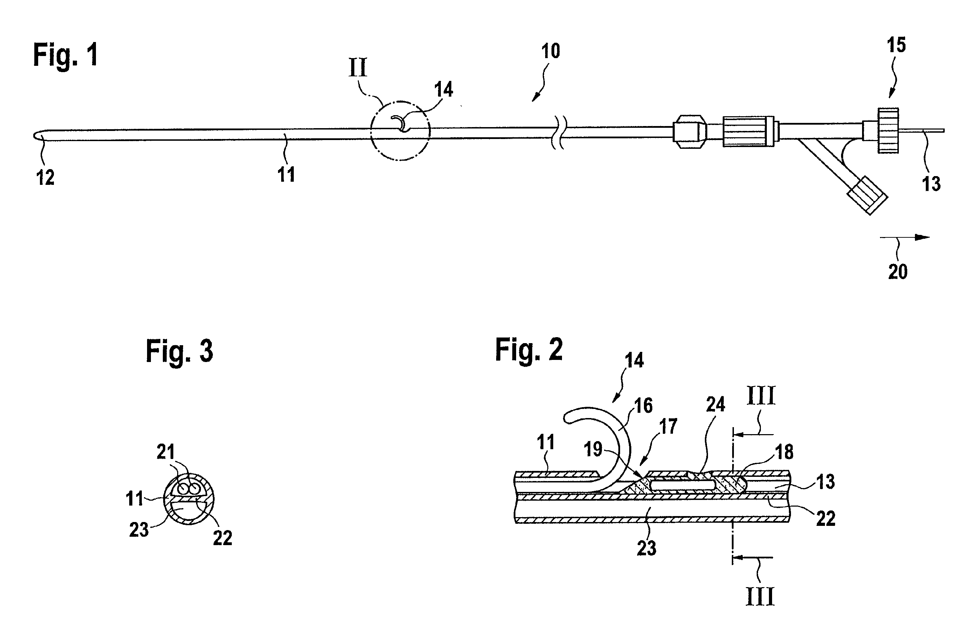

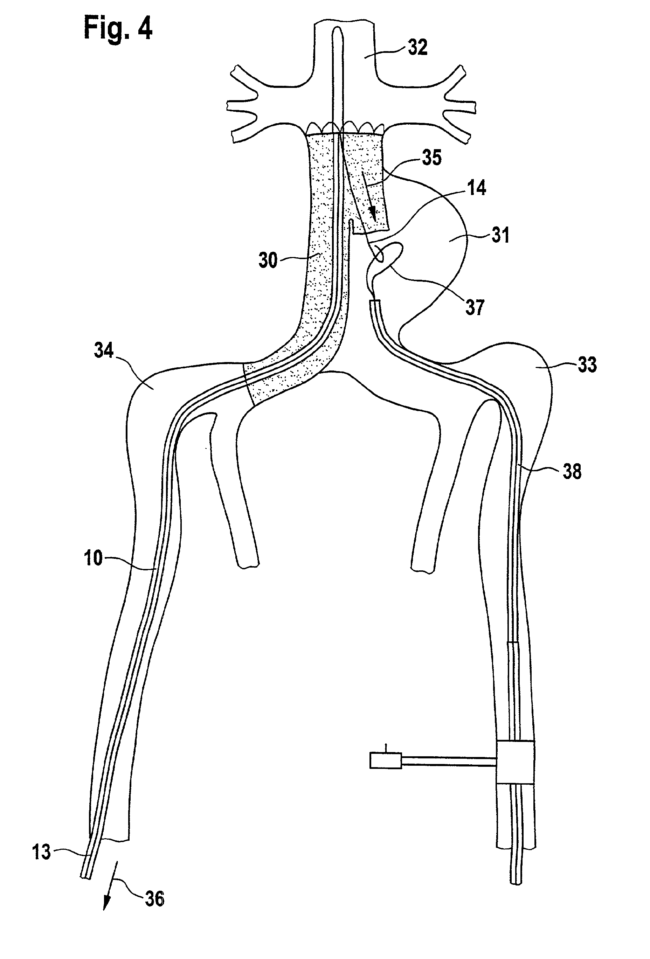

Fig. 1 zeigt eine Vorrichtung 10 zum Platzieren von Gefäßprothesen mit einem Katheterrohr 11, dessen Spitze 12 geschlossen und abgerundet ist. Im Inneren des Katheterrohrs 11 sind ein erster Führungsdraht 13 und ein zweiter Führungsdraht 14 geführt. Der erste Führungsdraht 13 ist länger als das Katheterrohr 11 und ragt über das äußere Ende 15 der Vorrichtung heraus. Der zweite Führungsdraht 14 ist deutlich kürzer als der erste Führungsdraht 13 und mit Abstand von der Spitze des ersten Führungsdrahts 13 mit diesem verbunden (nicht dargestellt), wobei im entspannten Zustand die beiden Drähte 13 und 14 einen spitzen Winkel einschließen, wie später an Fig. 4 noch näher beschrieben wird.

Der zweite Führungsdraht 14 weist eine hakenförmig gebogene Spitze 16 auf, wie insbesondere aus Fig. 2 ersichtlich ist. Diese hakenförmige Spitze 16 ragt in den Darstellungen der Fig. 1 und 2 durch eine seitliche öffnung 17 (Fig. 2) im Katheterrohr 11 nach außen. Wie Fig. 2 zeigt, ist unmittelbar benachbart zur seitlichen öffnung 17 ein Führungskörper 18 im Katheterrohr 11 angeordnet. Der Führungskörper 18 bildet eine Rampe 19, an der die Spitze 16 des zweiten Führungsdrahts 14 durch die öffnung 17 nach außen gleitet, wenn der erste Führungsdraht 13 im Katheterrohr 11 in Pfeilrichtung 20 (Fig. 1) zurückgezogen wird. Der Führungskörper 18 weist außerdem zwei in Fig. 3 ersichtliche Durchtrittsöffnungen 21 für die beiden Führungsdrähte 13 und 14 auf. Das gesamte Katheterrohr 11 ist außerdem durch eine Trennwand 22 längs in zwei Hälften geteilt, sodass eine weitere Kammer 23 entsteht. durch die ein weiterer Führungsdraht geführt werden könnte. Der Führungskörper 18 besteht aus Klebstoff, der durch eine zusätzliche öffnung 24 im Katheterrohr 11 eingebracht wird und diese öffnung 24 dabei mit ausfüllt, sodass eine formschlüssige Verbindung zwischen Führungskörper 18 und Katheterrohr 11 entsteht.

Fig. 4 demonstriert ein Anwendungsbeispiel einer erfindungsgemäßen Vorrichtung 10 zum Einsatz einer Aorta-Endoprothese 30 zur Behandlung eines Aneurysma 31 in der Bauchaorta 32. Das Aneurysma 31 liegt kurz vor der Verzweigung der Bauchaorta 32 in die linke und rechte Beckenarterie 33, 34. Aus diesem Grund muss die Gefäßprothese 30 insgesamt einen Y-förmigen Aufbau haben. Komplett ist eine solche Prothese jedoch nicht zu montieren. Aus diesem Grund wird die Gefäßprothese 30 aufgeteilt in die in Fig. 4 dargestellte Hauptprothese, die im Bereich der Spitze des schwarz eingezeichneten Pfeiles 35 um einen weiteren Schenkel, der hier nicht dargestellt ist, ergänzt wird. Zur Erleichterung des Einführens und Ansetzens dieses zusätzlichen Schenkels kann die erfindungsgemäße Vorrichtung 10 aus Fig. 1 mit Vorteil eingesetzt werden. Sie wird dazu entlang beispielsweise der rechten Beckenarterie 34 bis in die Bauchaorta 32 oberhalb des Aneurysma 31 eingeführt. Sie bietet dann eine gute Führung zum Platzieren der Gefäßprothese 30. Anschließend wird der erste Führungsdraht 13 in der Vorrichtung 10 in Pfeilrichtung 36 nach unten gezogen, wodurch der zweite Führungsdraht 14 aus der seitlichen öffnung 17 (Fig. 3) aus der Vorrichtung 10 in Pfeilrichtung 35 herausgleitet. Er verläuft dann entlang des Schenkelstumpfes der Prothese 30 und tritt aus dieser aus. Dadurch kann in die hakenförmige Spitze 16 (Fig. 3) des Führungsdrahts 14 ein schlingenförmiges Ende 37 eines durch die linke Beckenarterie 33 geführten Führungsdrahtes 38 eingefangen werden. Durch die Kopplung der Drähte 14 und 38 entsteht eine durchgehende Führung für den zusätzlichen Schenkel der Prothese 30, der damit leicht durch die linke Beckenarterie 33 bis zur Ankopplungsstelle geführt und mit der Prothese 30 verbunden werden kann.

Dieser Anwendungsfall der erfindungsgemäßen Vorrichtung ist jedoch nur beispielhaft. Die Vorrichtung 10 lässt sich mit Vorteil bei allen Arten von Sondierungen im Bereich von Gefäßverzweigungen einsetzen, beispielsweise auch zum Einsetzen von Kathetern oder dergleichen an Gefäßverzweigungsstellen zu diagnostischen Zwecken.

## Patentansprüche

1. Vorrichtung zur Sondierung von Gefäßabzweigungen, insbesondere zur Platzierung von Gefäßprothesen (30) an Gefäßverzweigungsstellen, mit einem Katheterrohr (11), in dem ein erster Führungsdraht (13), an dem im Abstand zu seiner Spitze ein zweiter Führungsdraht (14) befestigt ist, geführt ist, wobei die beiden Führungsdrähte (13, 14) im entspannnten Zustand einen spitzen Winkel einschließen und wobei das Katheterrohr (11) eine seitliche Öffnung (17) aufweist, aus der beim Zurückziehen der Führungsdrähte (13, 14) die Spitze (16) des zweiten Führungsdrahts (14) austreten kann, **dadurch gekennzeichnet, dass** auf der der Katheterrohrspitze abgewandten Seite der seitlichen öffnung (17) im Katheterrohr (11) ein Führungskörper (18) angeordnet ist, der eine zum öffnungsrand hinführende Rampe (19) bildet, die die Spitze (16) des zweiten Führungsdrahts (14) beim Zurückziehen der Führungsdrähte (13, 14) durch die seitliche öffnung (17) nach außen leitet.

2. Vorrichtung nach Anspruch 1, **dadurch gekennzeichnet, dass** die Spitze (16) des zweiten Führungsdrahts (14) hakenförmig gekrümmt ist.

3. Vorrichtung nach Anspruch 1 oder 2, **dadurch gekennzeichnet, dass** der Führungskörper (18) zwei Durchgangsöffnungen (21) zum Durchtritt der beiden Führungsdrähte (13, 14) aufweist.

4. Vorrichtung nach einem der Ansprüche 1 bis 3, **dadurch gekennzeichnet, dass** der Führungskörper (18) formschlüssig mit dem Katheterrohr (11) verbunden ist.

5. Vorrichtung nach einem der Ansprüche 1 bis 4, **dadurch gekennzeichnet, dass** der Führungskörper (18) aus einem Klebstoff gebildet ist.

6. Vorrichtung nach Anspruch 5, **dadurch gekennzeichnet, dass** der Klebstoff durch eine weitere seitliche öffnung (24) in der Wand des Katheterrohrs (11) einbringbar ist, wobei er diese öffnung (24) ausfüllt und so eine formschlüssige Verbindung mit dem Katheterrohr (11) eingeht.
